(19) Europäisches Patentamt

European Patent Office

Office européen des brevets

(11) Numéro de publication : **0 628 629 A1**

(12) **DEMANDE DE BREVET EUROPEEN**

(21) Numéro de dépôt : 94401296.2

(22) Date de dépôt : 09.06.94

(51) Int. Cl.[5] : **C12N 9/02,** // (C12N9/02, C12R1:89)

(30) Priorité : **11.06.93 FR 9307057**

(43) Date de publication de la demande : **14.12.94 Bulletin 94/50**

(84) Etats contractants désignés : **DE ES FR GB IT**

(71) Demandeur : **HELIOSYNTHESE S.A.**
**Centre d'Affaires Actimart,**
**Bureau 218,**
**1140 rue Ampère,**
**Pole d'Activité Les Milles**
**F-13795 Aix en Provence Cédex 03 (FR)**

(72) Inventeur : **Gudin, Claude**
**Le Rond Point,**
**36, Boulevard Mongin**
**F-13500 Martigues (FR)**
Inventeur : **Trezzy, Claudine**
**37, Domain des Vignes**
**F-13470 Le Rove (FR)**

(74) Mandataire : **Dubois-Chabert, Guy et al**
**Société de Protection des Inventions**
**25, rue de Ponthieu**
**F-75008 Paris (FR)**

(54) **Procédé de production et d'extraction de superoxyde-dismutases thermostables à partir d'une culture de micro-organismes photosynthétiques.**

(57) Le procédé de production et d'extraction de superoxyde-dismutases thermostables, consiste a) à cultiver dans la gamme de températures allant de 40°C à 80°C, dans un photoréacteur clos en matériau transparent à la lumière et thermiquement résistant dans ladite gamme, des micro-organismes thermophiles photosynthétiques aérobies, producteurs d'oxygène et à croissance exponentielle dans ladite gamme, ces micro-organismes étant suspendus dans un milieu de culture et choisis parmi les micro-algues et les cyanobactéries et b) à extraire du milieu de culture les superoxyde-dismutases thermostables fraîchement produites, par broyage cellulaire (4), ultrafiltration (10) et précipitation (12) sélective.

EP 0 628 629 A1

La présente invention a pour objet un procédé de production et d'extraction de superoxyde-dismutases stables thermiquement à partir d'une culture de micro-organismes photosynthétiques en suspension dans un milieu liquide. Le procédé permet une production intensive et contrôlée de superoxyde-dismutases (SOD) thermostables, à l'échelle industrielle. En particulier, ces SOD sont thermostables jusqu'à 80°C.

Les SOD sont des catalyseurs de la dismutation des ions superoxydes. Aussi, elles sont utilisées dans les phénomènes aigus de libération de radicaux superoxydes ou dans les phénomènes de vieillissement.

Ainsi, les SOD de l'invention sont destinées aux domaines pharmaceutique comme anti-inflammatoires (rhumatismes, arthrite), cosmétologique comme anti-vieillissements et pour la protection de la peau et des cheveux notamment contre les ultraviolets, agro-alimentaire comme protecteurs des huiles et corps gras, comme agents de régulation de la coloration, agricole comme engrais, ainsi que dans le domaine biomédical.

Les SOD sont constituées de deux sous-unités peptidiques liées entre elles. Elles sont classées en trois catégories selon la nature du métal entrant dans leur constitution : les SOD à Cu/Zn d'orign humaine ou animale, les SOD à Fe fréquentes chez les bactéries aérobies et les SOD à Mn.

Les SOD de l'invention font partie de la troisième catégorie. Les autres à Fe ou à Cu/Zn s'y trouvent accessoirement

La plupart des SOD actuellement produites et utilisées sont détruites lorsqu'elles sont soumises à un traitement thermique supérieur à 40°C comme par exemple la pasteurisation ou la stérilisation nécessitant des températures de 70°C environ. Ainsi, la SOD d'origine animale est détruite à partir de 37°C. Or, il est souhaitable d'utiliser en cosmétologie mais aussi dans les domaines pharmaceutique et biomédical des produits stérilisés.

En outre, une tenue à 50°C des SOD ou des extraits riches en SOD permettrait d'accélérer l'étude du vieillissement des produits cosmétologiques, dans des conditions économiques.

Aussi, des recherches récentes sont effectuées dans différents laboratoires pour produire des SOD thermostables.

Ainsi, le document (1) de A. Hosono et al. ("Superoxide-dismutase activity in the crude cell extract from *Streptococcus salivarius* sous-espèce *thermophilus* 3535" Anim Sci. Technol. Jpn., 62, 1991, p. 39-44) compare la production de SOD par sept souches de bactéries lactiques anaérobies et étudie la stabilité de ces activités enzymatiques en fonction du pH et de la température. La meilleure stabilité thermique est obtenue pour la SOD produite par *Streptococcus salivarius*, sous-espèce *thermophilus* qui reste stable lorsqu'elle est soumise à un traitement thermique de 60°C pendant 30 minutes mais commence à se dégrader pour un traitement thermique de 70°C.

Le document (2) de R. A. Hallewel et al. ("Thermostabilization of recombinant human and bovin Cu/Zn superoxide-dismutases by replacement of free cysteines", Biochem. and Biophys. Research Communications, vol. 181, n°1, 1981, p. 474-480) décrit l'augmentation de la thermostabilité de SOD humaine ou bovine obtenue par mutation sur des levures contenant le gène codant pour la SOD, lorsqu'on enlève un résidu de cystéine libre entrant dans la composition en acides aminés de la protéine enzymatique d'origine.

Dans le document (3) de K. B. Searcy et D. G. Searcy ("Superoxide-dismutase from the archaebacterium *Thermoplasma acidophilum*" Biochimica et Biophysica Acta, 670, 1991, p. 39-46), il est décrit la culture d'une bactérie aérobie à 59°C et à pH 1-2 produisant une SOD contenant du fer et du zinc présentant, après un traitement thermique à 100°C une activité enzymatique égale à 71% de celle obtenue après un traitement à 30°C, cette dernière étant en outre égale à celle mesurée avant tout traitement.

Les différentes SOD thermostables décrites dans ces documents sont des espèces hétérotrophiques consommatrices d'oxygène, ce qui conduit à une biosynthèse de SOD régulée par la concentration d'oxygène dissous dans le milieu de culture. Or, ces espèces consommatrices d'oxygène abaissent constamment le niveau de ce dernier qui devient alors un facteur limitant la croissance même de ces espèces, limitant ainsi la production de SOD.

Ainsi, les procédés de production de SOD thermostables connus ne permettent pas une production intensive et industrielle de SOD thermostables pouvant résister à des étapes de stérilisation à chaud.

L'invention a justement pour objet un nouveau procédé de production et d'extraction de superoxyde-dismutases thermostables utilisant des micro-organismes photosynthétiques, c'est-à-dire capables de transformer grâce à l'énergie solaire du dioxyde de carbone en biomasse avec comme principal sous-produit de cette transformation biochimique la production d'oxygène.

Ainsi, ces espèces photosynthétiques élèvent constamment le niveau d'oxygène dans le milieu de culture décuplant ainsi la production de SOD par les micro-organismes en réaction contre ce milieu fortement oxydant.

Aussi, l'invention a pour objet un procédé de production et d'extraction de superoxyde-dismutases thermostables, consistant a) -à cultiver dans la gamme de températures allant de 40°C à 80°C, dans un photoréacteur clos en matériau transparent à la lumière et thermiquement résistant dans ladite gamme, des micro-organismes thermophiles photosynthétiques aérobies, producteurs d'oxygène et à croissance exponentielle dans ladite gamme, ces micro-organismes étant en suspension dans un milieu de culture et choisis parmi les micro-algues et les cyanobactéries et b) - à extraire du milieu de culture les superoxyde-dismutases thermostables fraîchement produites.

Les micro-algues auxquelles s'applique l'invention sont de la classe des Chlorophyceae ou Rhodophyceae. A titre d'exemples de micro-algues utilisables dans l'invention, on peut citer les *Chlorella* telles que *Chlorella* vulgaris et *Chlorella saccharophila*, les *Dunaliella* telles que *Dunaliella salina*, les *Cyanidium* tels que *Cyanidium caldarium*.

Les cyanobactéries connues aussi sous le nom d'algues bleues produisent comme les micro-algues et à l'inverse des autres bactéries, de l'oxygène par photosyonthèse et des SOD à Mn et leur comportement en milieu oxydant, ainsi que leur culture sont très comparables à celles des micro-algues.

Comme cyanobactéries auxquelles s'applique l'invention, on peut citer les familles Chroococcacées, Stigonématacées, Mastigocladacées, Oscillatroriacées. A titre d'exemples, on peut citer les *Synéchococcus* tels que *Synéchococcus lividus et Synéchococcus elongatus* ; les *Synéchocystis* tels que *Synéchocystis minervae* ; les *Mastigocladus* tels que *Mastigocladus laminosus* ; les *Phormidium* tels que *Phormidium laminosus* ; les *Symploca* tels que *Symploca thermalis* ; les *Aphanocapsa* tels que les *Aphanocapsa thermalis* ; ou encore les *Fisherella*.

La culture des micro-organismes photosynthétiques se fait en ensoleillement naturel. Aussi, en vue d'augmenter la production de SOD en prolongeant la période de photosynthèse et donc de production d'oxygène, il est préférable d'effectuer la culture des micro-organismes en superposant à l'ensoleillement naturel un éclairage artificiel pouvant être maintenu pendant la nuit.

En outre, en vue d'augmenter la production de SOD, on enrichit avantageusement le milieu de culture en oxygène et/ou en gaz carbonique.

L'apport en $O_2$ et/ou $CO_2$ peut être réalisé en injectant ces gaz sous forme moléculaire ou d'air dans le photoréacteur. Par ailleurs, on peut augmenter la quantité d'$O_2$ dans le photoréacteur en limitant son évacuation du photoréacteur et/ou en le réinjectant dans le photoréacteur.

Afin d'améliorer la stabilité thermique des SOD produites, on effectue l'extraction de ces SOD dans la gamme de températures allant de 40°C à 80°C. En particulier, l'extraction est effectuée à la même température que celle utilisée pour la culture des micro-organismes.

Selon l'invention, la production des SOD thermostables et leur extraction sont réalisées en ligne et en continu.

De façon avantageuse, l'étape d'extraction des superoxyde-dismutases consiste à :

i) - séparer les micro-organismes du milieu de culture ;

ii) - broyer les micro-organismes obtenus en i) pour en extraire un liquide contenant des superoxyde-dismutases et d'autres protéines ;

iii) - concentrer ledit liquide ;

iv) - séparer ces autres protéines sélectivement par rapport aux superoxyde-dismutases.

Avantageusement, la concentration du liquide produit par les micro-organismes broyés est réalisée par microfiltration et la séparation des SOD des autres protéines est réalisée par ultrafiltration sélective. Une gamme de membranes (300 000, 100 000 et 30 000 daltons) permet de séparer les grosses protéines (comme la Rubisco) des phycobiliprotéines (100 000 à 200 000 daltons), et des petites protéines comme l'anhydrase carbonique. La SOD se retrouve dans la fraction des phycobiliprotéines qui sont ensuite précipitées par le sulfate d'ammonium. La SOD reste dans le surnageant.

La propriété thermostable des SOD produites par le procédé de l'invention n'est pas due uniquement au chauffage du milieu de culture entre 40°C et 80°C, mais au fait que ces SOD sont produites par des micro-organismes thermophiles qui ont leur optimum de croissance dans la zone thermophile de 40°C à 80°C. Ainsi, une souche de micro-organismes se développant en-dessous de ces conditions optimum de croissance produira une SOD généralement non thermostable.

Le chauffage du milieu de culture peut être réalisé en utilisant l'eau chaude provenant du thermalisme et/ou en utilisant des photoréacteurs clos ayant un rapport surface/volume favorable, c'est-à-dire supérieur à 10, dans des régions à fort ensoleillement.

Par matériau transparent résistant thermiquement entre 40°C et 80°C, il faut comprendre un matériau qui reste transparent à la lumière, sans s'opacifier ou se fragiliser lorsqu'il est utilisé dans cette gamme de températures. De préférence, le photoréacteur est réalisé en un matériau résistant à une température supérieure à 120°C. A titre d'exemples de matériaux, on peut citer le verre ou le polyméthacrylate de méthyle (Plexiglass[R]).

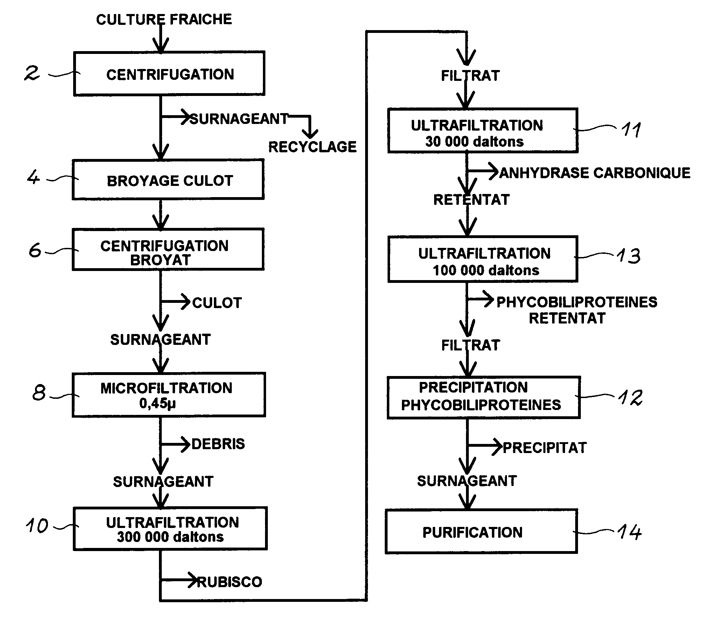

D'autres caractéristiques et avantages de l'invention ressortiront mieux de la description qui suit, donnée à titre illustratif et non limitatif en référence à l'unique figure qui illustre schématiquement les différentes étapes du procédé conforme à l'invention.

Le procédé de production de SOD thermostables entre 40°C et 80°C consiste tout d'abord à cultiver dans un photobioréacteur clos, et donc dans des conditions d'aérobie, des micro-algues ou des cyanobactéries thermophiles photosynthétiques, productrices d'oxygène. Le photoréacteur est en particulier celui décrit dans le document (4) FR-A-2 656 874, comportant des tubes transparents, dans lequel circule le milieu de culture contenant les micro-organismes, réalisés selon l'invention en verre. Ce photoréacteur peut en outre être équipé du dispositif de nettoyage automatique des tubes tel que décrit dans le document (5) FR-A-2 674 458, ainsi que du système "air lift" décrit dans le document (6) FR-A-2 685 344.

Le milieu de culture dans lequel sont suspendus les micro-organismes dépend de ces micro-organismes. Ainsi, la culture peut être réalisée sur eau douce ou eau de mer avec un pH allant de 1 à 8. En outre, ce milieu de culture est chauffé à une température choisie entre 40°C et 80°C qui est fonction des micro-organismes photosynthétiques cultivés. Cette température est choisie de façon à correspondre à la température de croissance optimum des micro-organismes thermophiles utilisés.

Dans le tableau I ci-après, on donne différents exemples de micro-organismes utilisables dans l'invention ainsi que leur température de croissance optimum, la nature de l'eau utilisée pour la culture et le pH du milieu de culture.

Pour les cyanobactéries, on utilise comme substrat nourricier le milieu BG11 dont la composition est donnée en particulier dans le document (7) ("Generic assignments, strain histories and properties of pure cultures of *Cyanobacteria*", de R. Rippka et al., Journal of General Microbiology, vol. 111, 1979, p. 4.

Pour *Dunaliella salina*, on utilise le milieu nutritif décrit dans le document (8) de Pick et al. ("Plant physiol.", 81, 1986, p. 92-96).

Pour *Cyanidium caldarium*, on utilise le milieu de Allen décrit dans la référence (9), (Allen, NB-1959, Arch. Mikrobiol. 32, p. 270-277).

Pour les *Chlorella*, on utilise le milieu BB décrit dans la référence (10), de Nichols HW et Bold HC (J. Phycol. 1, 1965, p. 34-38).

La production des micro-algues et des cyanobactéries se fait en condition naturelle d'éclairement et en particulier dans les zones climatiques à fort ensoleillement. Avantageusement cet éclairement est complété par un éclairage artificiel complémentaire, du type lumière du jour, comme dans une serre, pour prolonger la photopériode naturelle et la production d'oxygène photosynthétique, notamment lorsque le soleil a disparu, afin d'augmenter la production de SOD.

De plus, le milieu de culture est enrichi en oxygène et/ou en dioxyde de carbone, comme décrit dans le document (4) à l'aide d'un carbonateur et d'un système de récupération et de réinjection de l'oxygène fourni par les micro-organismes au cours de la photosynthèse, dans le milieu de culture.

Le temps de séjour des micro-organismes dans le milieu de culture dépend de leur constitution et varie entre 12 heures et 15 jours.

L'extraction en ligne des SOD thermostables est effectuée en phase exponentielle de croissance des micro-organismes, c'est-à-dire sur une culture fraîche.

Comme représenté sur la figure unique, l'extraction des SOD thermostables consiste tout d'abord à centrifuger, comme indiqué en 2, la culture notamment entre 10000g et 30000g pendant 30 min. Le surnageant ainsi produit peut être recyclé dans le photobioréacteur en vue d'une culture en continu.

Selon l'invention, la centrifugation 2 est effectuée de préférence en maintenant le milieu de culture à la température qu'il avait dans le photoréacteur.

Le culot issu de la centrifugation 2 est alors broyé, comme indiqué en 4, à l'aide d'une presse industrielle afin de subir un cycle de pressions-dépressions dans un homogénéisateur fonctionnant à une pression de $2.10^7$Pa. Le broyage est effectué notamment à chaud entre 40°C et 80°C.

On effectue ensuite, comme représenté en 6, une nouvelle centrifugation du broyat cellulaire obtenu dans une centrifugeuse continue tournant entre 10 000 g et 45 000 g pendant 1 h. Le culot issu de cette centrifugation 6 contient essentiellement des résidus cellulaires. Il peut être exploité pour les acides gras qu'il contient.

Le surnageant de la centrifugation 6 renferme la SOD thermostable ainsi que des phycobiliprotéines. Ces protéines ont un poids moléculaire allant de 120 000 à 240 000 daltons et un point isoélectrique allant de 4,5 à 5,11.

La centrifugation 6 est aussi effectuée à la température utilisée pour la croissance des micro-algues ou cyanobactéries.

Le surnageant est alors soumis à une microfiltration 8 à 0,45 µ puis à une première ultrafiltration 10 utilisant une membrane de filtration à seuil de coupure de 300 000 daltons pour séparer les grosses protéines, comme la Rubisco des moyennes protéines telles les phycobiliprotéines (100 000 à 200 000 daltons) et des petites protéines, comme l'anhydrase carbonique.

On effectue ensuite une nouvelle ultrafiltration 11 avec une membrane de 30 000 daltons pour séparer les petites protéines des moyennes protéines puis une troisième ultrafiltration 13 avec une membrane de 100 000 daltons pour séparer la majeure partie des phycobiliprotéines de la SOD.

Ces microfiltrations et cette ultrafiltration sont effectuées avantageusement à la température de culture des micro-organismes.

Le concentrat (ou filtrat) est recueilli et additionné de sulfate d'ammonium. La quantité de sulfate utilisée représente 10 fois le poids de concentrat recueilli.

Ce sulfate d'ammonium permet la précipitation 12 des phycobiliprotéines résiduelles.

Selon l'invention, cette précipitation 12 est effectuée à la température de culture des micro-organismes.

Le surnageant contenant les SOD thermostables concentrées est alors purifié comme indiqué en 14 par chromatographie en phase liquide ou gazeuse.

Ces SOD sont des macromolécules de 30 000 à 80 000 daltons à Mn avec un point isoélectrique de 4,2 environ.

Ces SOD peuvent alors être stérilisées ou pasteurisées en vue de leur utilisation dans les cosmétiques et les médicaments.

On donne ci-après des exemples de production de SOD thermostables.

## EXEMPLES 1 à 4

Dans les conditions décrites précédemment, on a cultivé trois espèces thermophiles respectivement *Synechococcus lividus ; Mastigocladus laminosus* et *Cyanidium caldarium* .

Les conditions de culture et les quantités de SOD produites exprimées par mg de matière sèche sont données dans le tableau II ci-après.

## CONTRE-EXEMPLES 1 et 2

Parallèlement, on a effectué la culture d'espèces mésophiles telles que *Porphyridium cruentum* et *Skeletonema costatum,* dans un réacteur identique à celui utilisé pour les espèces thermophiles ci-dessus et on a mesuré la quantité de SOD produite par ces espèces mésophiles. Les résultats sont aussi portés dans le tableau II.

La quantité de SOD est déterminée selon la méthode au Nitroblue Tetrazolium, noté NBT, ou la méthode à la Xanthine Oxydase, notée XO. Ces méthodes de mesure sont en particulier décrites dans la référence (11) de A.N. Michelson et al. ("Superoxides and superoxide-dismutases", Academic Press, 1977, p. 11).

Dans le tableau II, la quantité de SOD indiquée correspond à celle obtenue avant d'être soumise à un traitement thermique.

Les espèces des exemples 1 à 4 et contre-exemples $C_1$, $C_2$ ci-dessus ont été soumises à un broyage cellulaire comme décrit à l'étape 4. Ensuite, elles ont été soumises à un traitement thermique.

On constate que les espèces thermophiles ($Ex_1$ à $Ex_4$) cultivées dans les conditions décrites pré cédemment présentaient, après deux heures de chauffage à 50°C, une quantité de SOD représentant 100% de celle mesurée avant le traitement thermique.

En outre, après 30 minutes de chauffage à 80°C, on obtenait encore 100% de la quantité de SOD mesurée avant ce traitement thermique.

En revanche, après 30 minutes de chauffage à 60°C, les extraits cellulaires des espèces mésophiles des contre-exemples $C_1$ et $C_2$ ne présentaient que 50% de la quantité de SOD mesurée avant le traitement thermique.

Dans les exemples 1 à 4, les espèces thermophiles ont été cultivées sans enrichissement en oxygène et en dioxyde de carbone, c'est-à-dire avec un apport en dioxyde de carbone de 0,03% minimum nécessaire pour la photosynthèse.

En augmentant cet apport de $CO_2$, on constate une augmentation de la concentration en cellules photosynthétiques et donc de la concentration en oxygène ; en effet, une molécule de $CO_2$ fixée entraîne la production d'une molécule d'oxygène et donc d'une activité antiradicalaire. Ainsi, en doublant l'apport en $CO_2$, on peut doubler la quantité de SOD thermostables produites.

TABLEAU I

| ESPECE | EAU | TEMPERATURE °C | pH |
|---|---|---|---|
| *Cyanidium caldarium* | DOUCE | 40 à 50 | 1 à 5 |
| * *Chlorella vulgaris* | DOUCE | 40 à 50 | NEUTRE |
| * *Chlorella saccharophila* | DOUCE | 40 à 60 | NEUTRE |
| * *Dunaliella salina* | MER | 40 à 50 | NEUTRE |
| *Synechococcus lividus* | DOUCE | 50 à 80 | NEUTRE |
| * *Synechococcus elongatus* | DOUCE | 50 à 70 | NEUTRE |
| *Synechocystis minervae* | DOUCE | 40 à 60 | NEUTRE |
| *Mastigocladus laminosus* | DOUCE | 40 à 60 | NEUTRE |
| *Fisherella sp* | DOUCE | 40 à 60 | NEUTRE |
| * *Phormidium laminosus* | DOUCE | 40 à 60 | NEUTRE |
| * *Symploca thermalis* | DOUCE | 40 à 60 | NEUTRE |
| * *Aphanocapsa thermalis* | DOUCE | 40 à 60 | NEUTRE |

* Origine Russe

**TABLEAU II**

| CULTURE | | | | ACTIVITE SOD/mg matière sèche | |
|---------|--------|--------|-----|------------|-------------|
| **EX/CONTRE EXEMPLES** | **ESPECE** | **AGE (j)** | **T°C** | **XO en unité** | **NBT en unité** |
| EX 1 | *Synechococcocus lividus* | 3,5 | 40 | 6 | - |
| EX 2 | *Synechococcocus lividus* | 3,5 | 50 | 3 | - |
| EX 3 | *Mastigocladus laminosus* | 3,5 | 45 | - | 4 |
| EX 4 | *Cyanidium caldarium* | 15 | 40 | 3 | - |
| $C_1$ | *Porphyridium cruentum* | 3 | 15 à 30 | 4,3 | 10 |
| $C_2$ | *Skeletonema costatum* | 12 | 15 à 30 | 10 | 5,9 |

**Revendications**

**1.** Procédé de production et d'extraction de superoxyde-dismutases thermostables, consistant a) à cultiver dans la gamme de température allant de 40°C à 80°C, dans un photoréacteur clos en matériau transparent à la lumière et thermiquement résistant dans ladite gamme, des micro-organismes thermophiles photosynthétiques aérobies, producteurs d'oxygène et à croissance exponentielle dans ladite gamme, ces micro-organismes étant en suspension dans un milieu de culture et choisis parmi les micro-algues et les cyanobactéries et b) à extraire du milieu de culture les superoxyde-dismutases thermostables fraîchement produites.

**2.** Procédé selon la revendication 1, caractérisé en ce que l'extraction des superoxyde-dismutases est effectuée dans ladite gamme de températures.

**3.** Procédé selon la revendication 1 ou 2, caractérisé en ce qu'on enrichit le milieu de culture par de l'oxygène et/ou du gaz carbonique au cours de l'étape a).

**4.** Procédé selon l'une quelconque des revendications 1 à 3, caractérisé en ce que les micro-algues sont choisies parmi les *Cyanidium*, les *Chlorella* et les *Dunalliella*.

**5.** Procédé selon l'une quelconque des revendications 1 à 3, caractérisé en ce que les cyanobactéries sont choisies parmi les *Synéchococcus*, les *Synéchocystis*, les *Mastigocladus*, les *Phormidium*, les *Symploca*, les *Aphanocapsa* et les *Fisherella*.

**6** Procédé selon l'une quelconque des revendications 1 à 5, caractérisé en ce que l'étape a) est effectuée en lumière artificielle et/ou lumière solaire.

**7** Procédé selon l'une quelconque des revendications 1 à 6, caractérisé en ce que l'étape b) consiste à :
i) - séparer (2) les micro-organismes du milieu de culture ;
ii) - broyer (4) les micro-organismes obtenus en i) pour en extraire un liquide contenant des superoxyde-dismutases et d'autres protéines ;
iii) - concentrer (6, 8) ledit liquide ;
iv) - séparer (12) ces autres protéines sélectivement par rapport aux superoxyde-dismutases.

**8.** Procédé selon la revendication 7, caractérisé en ce que l'étape iv) comprend plusieurs étapes d'ultrafiltration sélective (10, 11, 13) et une étape de précipitation (12) avec du sulfate d'ammonium.

**9.** Procédé selon la revendication 7 ou 8, caractérisé en ce que l'étape i) est effectuée par centrifugation.

**10.** Procédé selon l'une quelconque des revendications 1 à 9, caractérisé en ce que le photoréacteur est en verre.

CULTURE FRAICHE

2 — CENTRIFUGATION

→SURNAGEANT
RECYCLAGE

4 — BROYAGE CULOT

6 — CENTRIFUGATION BROYAT

→CULOT
SURNAGEANT

8 — MICROFILTRATION 0,45μ

→DEBRIS
SURNAGEANT

10 — ULTRAFILTRATION 300 000 daltons

→RUBISCO

FILTRAT

ULTRAFILTRATION 30 000 daltons — 11

→ANHYDRASE CARBONIQUE
RETENTAT

ULTRAFILTRATION 100 000 daltons — 13

→PHYCOBILIPROTEINES RETENTAT
FILTRAT

PRECIPITATION PHYCOBILIPROTEINES — 12

→PRECIPITAT
SURNAGEANT

PURIFICATION — 14

**Office européen des brevets**

## RAPPORT DE RECHERCHE EUROPEENNE

Numero de la demande

EP 94 40 1296

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| Y | EP-A-0 437 393 (COMMISSARIAT A L'ENERGIE ATOMIQUE) 17 Juillet 1991<br>* le document en entier *<br>--- | 1-3,5-9 | C12N9/02<br>//(C12N9/02,<br>C12R1:89) |
| Y | PATENT ABSTRACTS OF JAPAN<br>vol. 014, no. 250 (C-0723)29 Mai 1990<br>& JP-A-02 069 181 (TADASHI MATSUNAGA) 8 Mars 1990<br>* abrégé *<br>--- | 1-3,5-9 | |
| A | HEALTH PHYSICS<br>vol. 53, no. 3 , Septembre 1987 , NEW YORK<br>pages 281 - 286<br>CONTER A. ET AL. 'Radiation stimulation during the early stationary growth phase in Synechococcus lividus and its correlation with photooxidative stress occuring before the stationary phase'<br>* le document en entier *<br>--- | 1,2,5,6 | |
| A | FR-A-2 635 531 (ECOLE CENTRALE DES ARTS ET MANUFACTURES) 23 Février 1990<br>* abrégé *<br>* page 5, ligne 11 - ligne 33 *<br>* page 10 - page 17 *<br>--- | 1-4,6,10 | **DOMAINES TECHNIQUES RECHERCHES (Int.Cl.5)**<br><br>C12N<br>C12R |
| A | EP-A-0 218 480 (SUNTORY LIMITED) 15 Avril 1987<br>* abrégé *<br>* page 7, ligne 6 - ligne 36 *<br>--- | 1,3,7-9 | |
| A | EP-A-0 533 942 (HAGIWARA Y.) 31 Mars 1993<br>* page 5, ligne 40 - ligne 52 *<br>* page 6, ligne 6 - ligne 21 *<br>---<br>-/-- | 1,5,6 | |

Le présent rapport a été établi pour toutes les revendications

| Lieu de la recherche | Date d'achèvement de la recherche | Examinateur |
|---|---|---|
| LA HAYE | 16 Septembre 1994 | Gac, G |

Office européen
des brevets

**RAPPORT DE RECHERCHE EUROPEENNE**

Numero de la demande

EP 94 40 1296

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | BIOCHIM. BIOPHYS. ACTA vol. 438, no. 2 , 8 Juillet 1976 pages 380 - 392 LUMSDEN ET AL. 'Purification and physicochemical properties of superoxide dismutase from two photosynthetic microorganisms' * le document en entier * ----- | 1,2 | |
| | | | **DOMAINES TECHNIQUES RECHERCHES (Int.Cl.5)** |

Le présent rapport a été établi pour toutes les revendications

| Lieu de la recherche | Date d'achèvement de la recherche | Examinateur |
|---|---|---|
| LA HAYE | 16 Septembre 1994 | Gac, G |